# EUROPEAN PATENT APPLICATION

(11) **EP 3 593 804 A1**
(43) Date of publication of application: **15.01.2020**
(21) Application number: 18183558.8
(22) Date of filing: 13.07.2018
(51) Int. Cl.: A61K 31/714, A61P 25/30

(54) **MECOBALAMIN FOR RELIEVING EMERGENCY DRUG POISONING**

(71) Applicant: Cheng, En-Che, 830 Kaohsiung City (TW)
(72) Inventor: Cheng, En-Che, 830 Kaohsiung City (TW)
(74) Representative: Cabinet Chaillot

(57) **Abstract**

Disclosed is a Mecobalamin(10) medicine given to a patient to relieve a patient's emergency drug addiction symptoms and pains before performing a subsequent poisoning pathway treatment(20) to block a drug to achieve better treatment effect, when the patient is suffering a drug withdrawal.

## Description

### BACKGROUND OF THE INVENTION

### Field of the Invention

The present invention relates to Mecobalamin for relieving emergency drug poisoning, and more particularly to a Mecobalamin medicine given to a patient when suffering a drug withdrawal for relieving the patient's emergency drug addiction symptoms and pains and then performing a subsequent poisoning pathway treatment to block a drug in order to achieve a better treatment effect.

### Description of the Related Art

U.S. Patent No. 10004781 invented and filed by the inventor of the present invention is disclosed to block the poisoning pathway of a drug (such as heroin or morphine), and it relates to a treatment prescription. However, when a patient is suffering a drug withdrawal, the patient needs to relieve emergency drug addiction symptoms and soothe the pains before a subsequent poisoning pathway treatment is performed. At present, there is no effective medicine to relieve the patient's emergency drug addiction symptoms and pains. Obviously, the effect of the poisoning pathway treatment requires further improvements. In view of the aforementioned drawbacks, the inventor of the present invention conducted research and experiment and finally developed a Mecobalamin medicine which is an active coenyzme B12 capable of supplying methyl groups to human bodies for transmethylation and repairing damaged nerve fibers, and the mecobalamin medicine is applicable of relieving symptoms including macrocytic anemia, megaloblastic anemia, pernicious anemia, and vitamin B12 deficiency. For emergency drug poisoning, the mecobalamin medicine has the effect of relieving the patient's drug addiction symptoms and pains. The inventor of the present invention believes that a patient's drug addiction symptoms and pains can be relieved with good treatment effect by giving the Mecobalamin medicine to the patient before a poisoning pathway treatment, whenever the patient is suffering a drug withdrawal.

### SUMMARY OF THE INVENTION

Therefore, it is a primary objective of the present invention to provide a medicine capable of relieving a patient's drug addiction symptoms and pains before the poisoning pathway treatment for blocking the drug is performed.

The present invention is characterized in that the Mecobalamin medicine given to a patient to relieve the patient's drug addiction symptoms and pains before the poisoning pathway treatment for blocking the drug poisoning has better treatment effect.

### BRIEF DESCRIPTION OF THE DRAWINGS

Fig. 1 is a flow chart of an application in accordance with a preferred embodiment of the present invention; and
Fig. 2 is a flow chart of applying a medicine containing Biperiden Hcl in accordance with a preferred embodiment of the present invention.

### DETAILED DESCRIPTION OF THE PREFERRED EMBODIMENTS

The technical characteristics, contents, advantages and effects of the present invention will be apparent with the detailed description of a preferred embodiment accompanied with related drawings as follows.

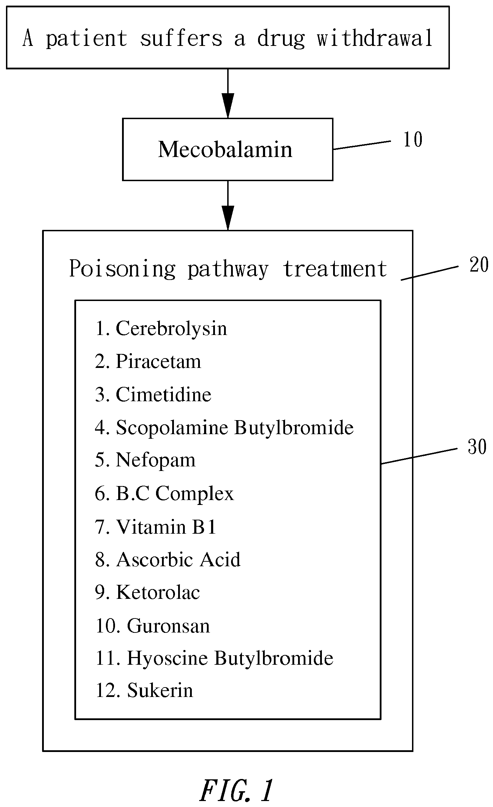

With reference to Fig. 1, the medicine of Mecobalamin 10 of the present invention is applied to relieve a patient's emergency drug addiction symptoms and soothe the patient's pains before performing a subsequent poisoning pathway treatment, whenever the patient is suffering a drug withdrawal. The Mecobalamin 10 (with a dose of 250∼500mg/ml) is given to the patient to relieve a patient's emergency drug addiction symptoms and soothe the patient's pains when the patient is suffering a drug withdrawal, and then a subsequent poisoning pathway treatment is performed. Wherein, the poisoning pathway treatment 20 adopts a drop prescription 30 and the treatment as disclosed in U.S. Pat. No. 10004781, and the drop prescription 30 is given to the patient, and the drop prescription 30 comprises a plurality of ingredients consisting of Cerebrolysin, Piracetam, Cimetidine, Scopolamine Butylbromide, Nefopam, B.C Complex, Vitamin B1, Ascorbic Acid, Ketorolac, Guronsan, Hyoscine Butylbromide, and Sukerin.

The Mecobalamin 10 of the present invention is given to the patient when the patient is suffering a drug withdrawal, so as to relieve the patient's drug addiction symptoms and soothe the patient's pains. This invention improves the patient's symptoms and pains significantly.

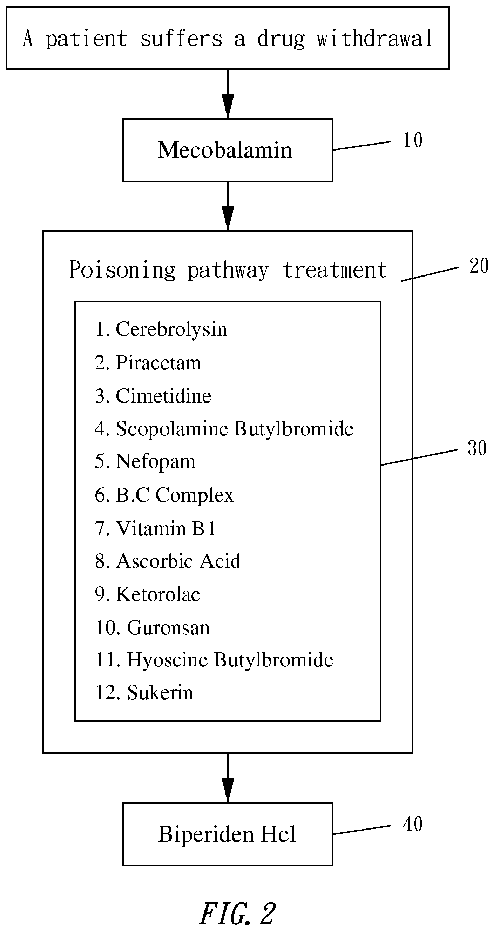

In FIG. 2, after the patient has received the aforementioned poisoning pathway treatment 20 to soothe the patient's brain nerve disorder produced after the treatment and enable the patient to recover his/her consciousness to a normal physiological condition quickly. After the poisoning pathway treatment 20, a Biperiden Hcl 40 (with a dose of 10mg/ml) is given to the patient, so that the patient can recover his/her consciousness to a normal physiological condition quickly.

When the patient is suffering a drug withdrawal, the Mecobalamin 10 of the present invention is given to the patient to relieve the patient's drug addiction symptoms and soothe the patient's pain. The invention can improve the symptoms and pains significantly. In addition, the patient can recover his/her consciousness to a normal physiological condition quickly after the poisoning pathway treatment 20 if the Biperiden Hcl 40 is given to the patient. The use of Biperiden Hcl 40 improves the patient's physiological conditions significantly.

According to one embodiment of the invention, the Mecobalamin for relieving emergency drug poisoning, is being used for preparing a medicine for relieving emergency drug addiction symptoms and pains before performing a subsequent poisoning pathway treatment, when a patient is suffering a drug withdrawal.

According to one embodiment of the invention, the poisoning pathway treatment prepares a drop prescription comprising a plurality of ingredients consisting of Cerebrolysin, Piracetam, Cimetidine, Scopolamine Butylbromide, Nefopam, B.C Complex, Vitamin B1, Ascorbic Acid, Ketorolac, Guronsan, Hyoscine Butylbromide, and Sukerin.

According to another embodiment of the invention, the Mecobalamin for relieving emergency drug poisoning further comprises a Biperiden Hcl medicine which is given to the patient after the poisoning pathway treatment.

According to another embodiment of the invention, the Mecobalamin is given to the patient with a dose of 250∼500mg/ml.

According to another embodiment of the invention, the Biperiden Hcl is given to the patient with a dose of 10mg/ml.

In summation of the above description, the present invention herein enhances the performance than the conventional structure and further complies with the patent application requirements and is submitted for patent application. While the invention has been described by means of specific embodiments, numerous modifications and variations could be made thereto by those skilled in the art without departing from the scope and spirit of the invention set forth in the claims.

## Claims

1. A Mecobalamin(10) for relieving emergency drug poisoning, being used for preparing a medicine for relieving emergency drug addiction symptoms and pains before performing a subsequent poisoning pathway treatment(20), when a patient is suffering a drug withdrawal.

2. The Mecobalamin(10) for relieving emergency drug poisoning according to claim 1, wherein the poisoning pathway treatment(20) prepares a drop prescription(30) comprising a plurality of ingredients consisting of Cerebrolysin, Piracetam, Cimetidine, Scopolamine Butylbromide, Nefopam, B.C Complex, Vitamin B1, Ascorbic Acid, Ketorolac, Guronsan, Hyoscine Butylbromide, and Sukerin.

3. The Mecobalamin(10) for relieving emergency drug poisoning according to claim 1, further comprising a Biperiden Hcl(40) medicine which is given to the patient after the poisoning pathway treatment(20).

4. The Mecobalamin(10) for relieving emergency drug poisoning according to claim 2, further comprising a Biperiden Hcl(40) medicine which is given to the patient after the poisoning pathway treatment(20).

5. The Mecobalamin(10) for relieving emergency drug poisoning according to claim 1, wherein the Mecobalamin(10) with a dose of 250∼500mg/ml is given to the patient.

6. The Mecobalamin(10) for relieving emergency drug poisoning according to claim 3, wherein the Biperiden Hcl(40) with a dose of 10mg/ml is given to the patient.

7. The Mecobalamin(10) for relieving emergency drug poisoning according to claim 4, wherein the Biperiden Hcl(40) with a dose of 10mg/ml is given to the patient.
